# EUROPEAN PATENT APPLICATION

(11) **EP 3 097 847 A1**
(43) Date of publication of application: **30.11.2016**
(21) Application number: 16170006.7
(22) Date of filing: 17.05.2016
(51) Int. Cl.: A61B 5/00, A61F 5/56, A61B 5/024, A61B 5/145, A61M 21/00

(54) **WEARABLE DEVICE AND SYSTEM FOR STOPPING AIRWAY DISORDERS INCLUDING SUCH A WEARABLE DEVICE**

(30) Priority: 28.05.2015 EP 15169605
(71) Applicant: Nitetronic Holding Limited, Grand Cayman KY1- 1112 (KY)
(72) Inventor: Edler von Janecek, Hubertus, Shanghai 200120 (CN); Herrnsdorf, Johannes, 58313 Herdecke (DE)
(74) Representative: Banse & Steglich

(57) **Abstract**

The invention relates to a wearable device (6, 6a, 6b, 6c) for detecting body characteristics and for use in an airways disorder alleviating system, comprising:
- a device housing ;
- a means (7) for attaching the device housing to a body part of a sleeping individual (2);
- one or more sensors (63-67) configured to detect body characteristics and
- a communication unit (61) configured to communicate at least one of information of body characteristics and a respiratory disorder event information indicating that a respiratory disorder event is ongoing or upcoming to an external device.

## Description

### Technical field

The present invention relates to systems, particularly head supports, for suppressing and/or stopping a respiratory disorder event. Particularly, the invention relates to methods for sensing and detecting body characteristics of a sleeping individual.

### Related art

A head support for stopping snoring of a sleeping individual is known by document US 14/358625, wherein the head support has a head resting surface for the head of a sleeping individual to rest on. The head support has an active layer with an arrangement of neighboring deforming elements for controlling the height of the head support section by section. To detect a position of the head resting on the head resting surface a sensing layer is provided which is arranged between the head resting surface and the active layer.

During sleep of an individual, pathologic effects such as snoring and/or sleep apnea can reduce oxygen absorption which may be dangerous to health of the sleeping individual. It has been found that moving head or body of the sleeping individual during snoring or during an ongoing sleep apnea event may immediately resolve the snoring or sleep apnea event, respectively, and urges the individual to continue normal breathing.

From prior art, various approaches are known to detect irregularities in respiration of a sleeping individual due to sleep apnea and trying to actively move the body and/or the head of the sleeping individual.

In document WO 2014/114438 A1, a sleeping pillow for alleviating a sleep apnea is disclosed. The sleeping pillow has an adjusting means system which can be actuated for changing the position of a head resting on the sleeping pillow by way of a control device and which has sensor means for detecting the position of the head. The control device is configured so that in addition sensor means signals from further sensor means for detecting respiratory activities of a person can be detected. In order to detect a sleep apnea of a person using the sleeping pillow, the further sensor means comprise sensors for detecting a breathing frequency or breathing movements of the chest of the person or sensors for detecting the nose dynamic pressure of a person. The adjusting system means systems can be actuated with respect to the sensor means signals of the further sensor means to alleviate or to avoid a sleep apnea event.

From document DE 10 2009 039 915 A1, an apparatus for the detection and signaling of the sleep-relating breathing dysfunction of an individual is known. Therein, it is described that a sensor is fixated at the head of an individual so that mechanical inertia at the head of the individual can be measured and analyzed to obtain head alignment information, breathing information about a breathing behavior and snoring information about the snoring behavior of the individual.

Document CN 103 222 909 A discloses a pillow with air chambers. Between the surfaces, a sensor is provided which is connected to a processor. The processor is configured to collect a sleep respiratory rate of a user. The sensor is configured as a sound sensor for acquiring the number of breaths, breath frequency rate and other sleep information from the user.

Document CN 102 743 246 A discloses an inflatable pillow with air chambers for changing the position of a head of an individual resting on the pillow. When a sleep apnea occurs, the individual's head is moved by locally actuating the air chambers of the pillow. Detection of the sleep apnea may be performed by means of a wrist assembly for detecting the patient's pulse and blood oxygen concentration, means for detecting a sleeping position or by means of a belly assembly for detecting a swing and abdomen abdominal breathing gas flow component.

Document CN 1557270 A discloses a pillow with inflatable air chambers which are controlled depending on an input of a respiratory sensor signal.

Document KR 100711701 B1 discloses a pillow for preventing a sleep-related breathing disorder. The pillow has a number of chambers in which a pressure can be regulated. By detecting the characteristics of pressure in each of the chambers, it can be estimated whether a breathing disorder is caused by comparing the pressure of each chamber with control data indicating an optimal pressure pattern data. The pressure of the air chambers is regulated, if a breathing disorder is detected.

It is therefore an object of the present invention to provide a robust and reliable detection of an airway disorder during sleep of an individual and to move a head of a sleeping individual depending on the detection of an airway disorder event.

While a detection of snoring as a respiratory disorder can be easily detected using microphones, particularly for detecting an upcoming or ongoing airways disorder event, such as a sleep apnea, cannot be remotely sensed. It is therefore a further object of the present invention to provide a means to measure further body characteristics which allow a monitoring of body functions and to provide this information to an airways disorder alleviating device.

### Summary of the invention

These and other objects can be resolved by the wearable device for detecting body characteristics according to claim 1 and the airways disorder alleviating system and head support according to the further independent claims.

Further embodiments are indicated in the dependent subclaims.

According to a first aspect a wearable device for detecting body characteristics and for use in an airways disorder alleviating system is provided, comprising:
- a device housing ;
- a means for attaching the device housing to a body part of a sleeping individual;
- one or more sensors configured to detect body characteristics and
- a communication unit configured to communicate at least one of information of body characteristics and a respiratory disorder event information indicating that a respiratory disorder event is ongoing or upcoming to an external device.

The above wearable device allows to detect body characteristics of a sleeping individual whose head rests on a head support. The wearable device is convenient to wear at a body part as it may be located at positions which are comfortable for the sleeping individual and since it abuts to the skin of the body part of the individual it allows to continuously monitor body characteristics needed for detecting an upcoming and/or ongoing airways disorder event, such as a snoring or a sleep apnea event.

The body characteristics can be analyzed in the wearable device or sensed signals can be communicated to a control unit of a head support in which the body characteristics are analyzed. The body characteristics are processed to detect an upcoming and/or ongoing respiratory disorder event. The wearable device can be worn close at the head or neck of the sleeping individual and allows to not only detect snoring noise, but also further body characteristics, such as airways airflow, airflow humidity, airflow alcohol level, blood oxygen saturation, the heart rate and the orientation of the head on a head support (side or supine alignment in a lying position of the individual). The information about the body characteristics can be provided to a control unit which analyzes the body characteristics detected and which can decide whether and how a movement of the head of the sleeping individual shall be performed to suppress or stop the respiratory disorder event.

Furthermore, the information can be transmitted to a simple monitoring device to monitor body characteristics during a sleep of the individual.

Furthermore, the means for attaching may be configured to allow the device housing to be attached to at least one of an upper side of the head of the sleeping individual, a forehead of the sleeping individual, a neck of the sleeping individual, a location between the nose and the mouth, and a chest of the sleeping individual, wherein the means for attaching particularly comprise at least one of a belt, a strap and a stripe.

It may be provided that the information of body characteristics comprises at least one of:
- a snoring noise,
- vibrations and/or an orientation of the device housing,
- a body temperature of the sleeping individual,
- a blood oxygen level and/or a heart rate,
- airflow humidity,
- airflow alcohol level, and
- a pressure sensor related to a respiratory activity of the sleeping individual.

Particularly, the one or more sensors may comprise one or more of:
- a microphone configured to detect a snoring noise,
- an inertia sensor configured to detect vibrations and/or an orientation of the device housing,
- a temperature sensor configured to detect the body temperature of the sleeping individual,
- a blood sensor configured to detect a blood oxygen level and/or a heart rate,
- a pressure sensor configured to detect a respiratory activity of the sleeping individual;
- a humidity sensor configured to detect an airflow humidity;
- an alcohol sensor configured to detect an airflow alcohol level.

Particularly, the inertia sensor in combination with one of the pressure sensor, the blood sensor and the microphone can be advantageously used with a head support which may interact with the head or body of the sleeping individual. The detection of the head orientation in combination with body characteristics in a single device makes the wearable device easy to use, especially in combination with the external device configured to move head or body of the sleeping individual to stop or to suppress respiratory disorder event.

Furthermore, a pressure sensor may be connected via a nasal air pipe to the nose of the sleeping individual.

According to an embodiment an inertia sensor is provided to detect an orientation of the head or body of the sleeping individual wherein the communication unit communicates an information about the orientation to an external device. The inertia sensor allows to provide the orientation of the head or the body as an additional information which allows to further evaluate other body characteristics.

According to a further aspect, a head support is provided, comprising:
- a resting surface for a head of a sleeping individual to rest on;
- an active layer configured to allow a separate deformation of the resting surface in sections;
- a control unit configured to receive at least one of information of body characteristics and to determine a respiratory disorder event information indicating that a respiratory disorder event is ongoing or upcoming based on the at least one of information of body characteristics and to actuate the active layer depending on the at least one of information of body characteristics.

Furthermore, the control unit may be configured to receive an information about the orientation of the head or the body of the sleeping individual as the at least one of information of body characteristics and to actuate the active layer depending on the information about the orientation of the head or the body.

Moreover, the control unit may be configured to receive an information about a breathing activity of the sleeping individual as the at least one of information of body characteristics and to actuate the active layer depending on the information about the breathing activity.

According to a further aspect, an airways disorder alleviating system is provided comprising the above mentioned wearable device and the above head support.

### Brief description of the drawings

Embodiments are described in more detail in conjunction with the accompanying drawings in which:
- Figure 1: schematically shows an airways disorder alleviating system;
- Figure 2: schematically shows possible locations for attachment of the wearable device;
- Figure 3: schematically shows a further possible location for attachment of the wearable device including a nasal airpipe;
- Figure 4: schematically shows a further possible location for attachment of a wearable device close to the nose of the sleeping individual;
- Figure 5: schematically shows a wearable device as a block diagram; and
- Figures 6a and 6b: exemplarily illustrate the use of the wearable device to detect the orientation of the head of the sleeping individual.

### Description of embodiments

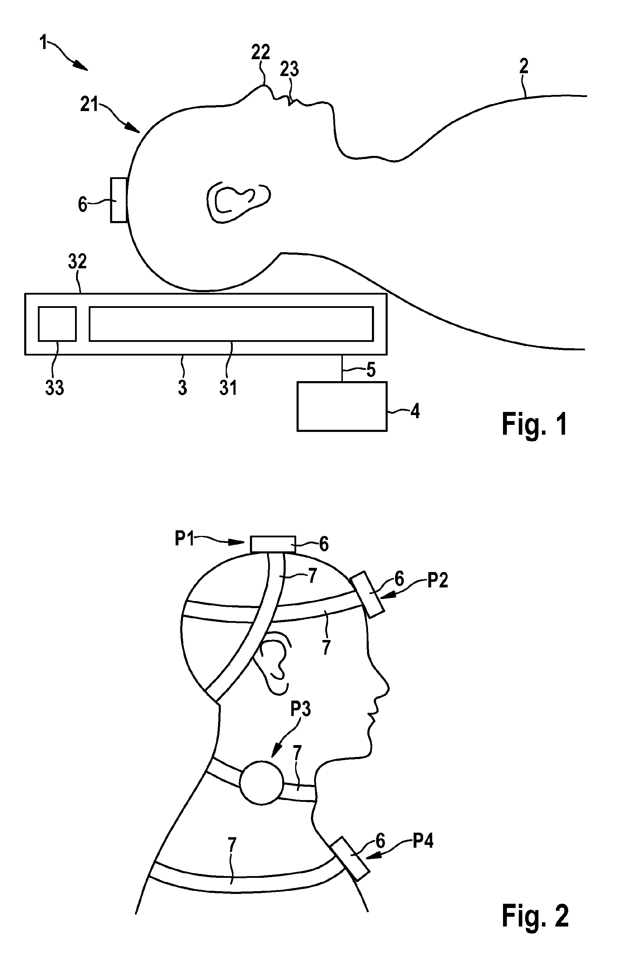

Figure 1 shows an airways disorder alleviating system 1 which is configured to stop an ongoing and/or upcoming airways disorder event of a sleeping individual 2. An airways disorder event is any kind of respiratory irregularity, such as snoring or a sleep apnea event.

The system 1 includes a head support 3, such as a pillow, on which a head 21 of the individual 2 rests while the individual is sleeping. The head support 3 may include a variety of components which include an active layer 31 which allows a separate deformation of a resting surface 32 of the head support 3 in sections so that the height of the resting surface 32 can be individually set. The active layer 31 may include deformation elements (not shown) which are closely neighbored or distanced from each other to allow that the height of the resting surface 32 of the head support 3 is changed in these sections on which the head of the sleeping individual 2 rests and/or in one or more sections neighbored thereto to change the orientation and/or position of the head 21 in a predetermined manner.

To identify the deformation elements which need to be actuated to move the head of the sleeping individual 2, the position of the head 21 can be detected by any kind of position detection means 33 in the head support 3. Alternatively, position detection means can be located external to the head support 3.

A control unit 4 may be provided which may be located external from the head support 3 and connected therewith via an interconnection 5. In alternative embodiments, the control unit 4 can be arranged internal of the head support 3.

At the body of the sleeping individual 2, a wearable device 6 may be attached which is configured to detect one or various body characteristics and to communicate the body characteristics continuously to the control unit 4. The transferring of information of body characteristics may be wireless or cable-bound, however, a wireless connection between a control unit 4 and the wearable device 6 is preferred to allow the sleeping individual to freely move on their head support. Suitable communication protocols for a transmission to the control unit 4 may be WiFi, Bluetooth or the like. Furthermore, an optical data transmission via LiFi using invisible light is preferred as electromagnetic radiation for data transmission can be reduced.

Figure 2 shows alternatives for arranging the wearable device 6 on the body of the individual 2. Possible locations for attaching the wearable device 6 are on the upper side of the head (position P1), on the forehead (position P2), on a neck (position P3) and on the chest (position P4). The wearable device 6 can be attached on the body by means of belts, straps or stripes 7 which may be elastic to securely attach the wearable device 6 at the body of the sleeping individual so that a displacement of the wearable device 6 is prevented. The materials of the belts, straps or stripes 7 may comprise plastics, rubber and/or metal. Particularly, the attachment is made so that one surface of the wearable device 6 abuts the skin of the sleeping individual 2 which facilitates detection of some body characteristics.

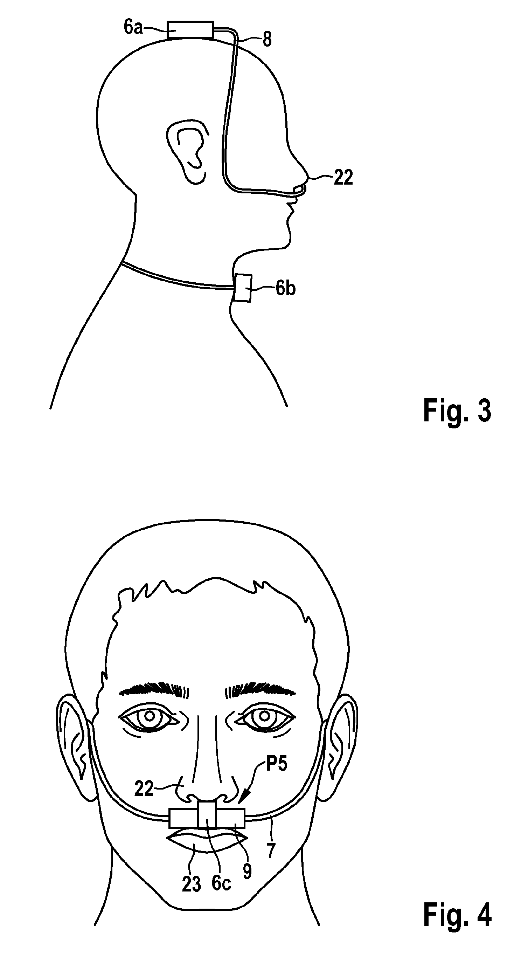

Further, combinations of multiple wearable devices 6 having different kind of sensors and which are arranged on different positions P1, P2, P3, P4, P5 are possible. For instance, the embodiment of Figure 3 shows the arrangement of a first wearable device 6a at an upper head or side of the head which is connected by means of a nasal air pipe 8 with a nose 22 of the sleeping individual 2 so that a pressure change in the air pipe 8 resulting from the breathing of the individual can be detected by a corresponding pressure sensor in the wearable device 6.

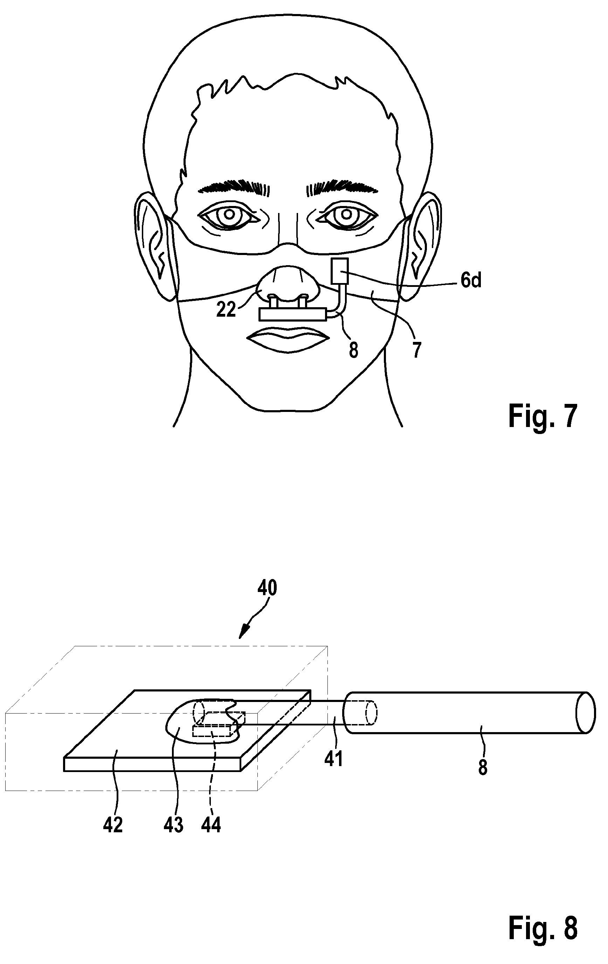

Another embodiment is shown in Figure 7 wherein another wearable device 6d is worn close to preferably aside a nose 22 of the sleeping individual. To hold the wearable device 6d belts, straps or stripes 7 may extend between the ears and are held in position by forming a loop around the head or by an attachment on the ears. The wearable device 6d may include the pressure sensor 40 among none, one or more other sensors.

As shown in Figure 8 in more detail, the pressure sensor 40 as used in the embodiments of Figures 2, 3, 4, and 7 may be connected with the nose airways of the sleeping individual via the nasal air pipe 8. The nasal air pipe 8 may be disposably connected to a first end of a tube 41 extending from the wearable device 6, 6a, 6b, 6c, 6d, wherein a second end of the tube 41 has an opening arranged close to a pressure sensor unit 44 fixated on a substrate 42. The tube 41 is arranged parallel to the sensing surface of the pressure sensor unit 44 and attached thereon by means of a glob-top 43 (e.g. epoxy resin) which is a piece of glue which allows a pressure change in the interior of the tube 41 to be propagated to and detected by the sensing surface of the pressure sensor unit 44. This arrangement allows to use disposable nasal air pipes 8 which finds more acceptance due to hygienical reasons. The attachment of the tube 41 on the pressure sensor unit 44 by means of a glob-top allows a flexible connection between the tube 41 and the pressure sensor unit 44 which may better accept movements which may occur between the pressure sensor 40 and the nasal air pipe 8 due to movement of the sleeping individual 2.

Furthermore, a second wearable device 6b including a vibration sensor can be alternatively or additionally arranged at the neck of the sleeping individual 2 so that a vibration can be detected which can be used to determine that a snoring detected by microphones arranged remote from the head of the sleeping individual originates from the individual on the head support 3.

Furthermore, a third wearable device 6c or a part, i.e. a sensor, thereof, can, as shown in Figure 4, alternatively or additionally be arranged close to the nose 22 of the sleeping individual 2, particularly between nose 22 and mouth 23 of the sleeping individual 2. This may have the advantage that airflow through both the nose 22 and the mouth 23 can be detected with the same sensor unit, such as an airflow sensor.

The third wearable device 6c can include a microphone to acoustically measure the airflow, a pressure sensor to measure pressure variations caused by breathing or a temperature sensor to measure nose airflow by means of temperature changes.

The third wearable device 6c can include a carrier plate 9 which can be made of soft plastic, rubber, textile or foam to lie closely on the skin at a location (position P5) between nose 22 and mouth 23 so that the breathing air can flow along the plate 9.

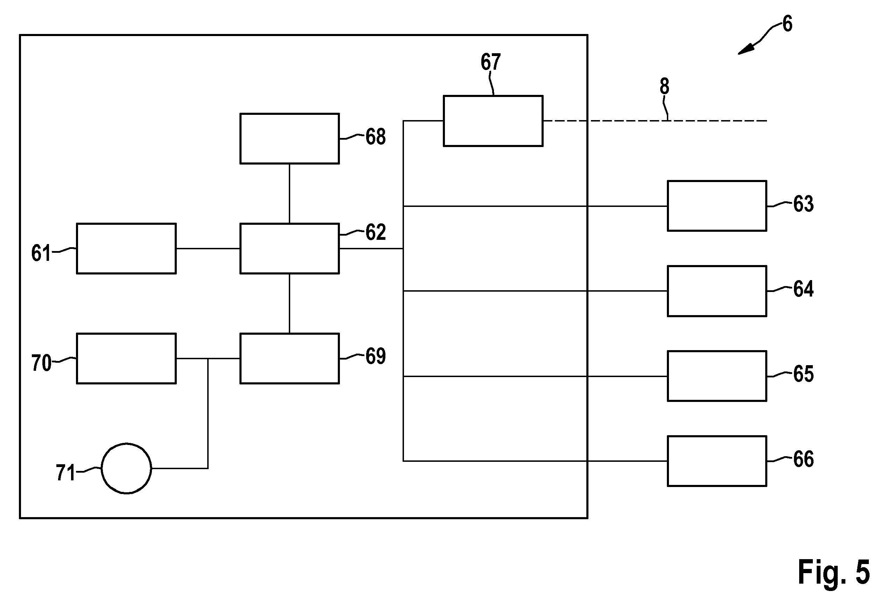

Figure 5 shows a block diagram illustrating the functionality of the wearable device 6 as described before. The wearable device 6 may have a communication unit 61 to preferably wirelessly communicate information of body characteristics to the control unit 4.

Furthermore, a controller 62 is provided which is connected to one or a combination of sensors 63-67 to obtain sensing signals therefrom. Just for illustration the wearable device 6 is shown with a number of sensors 63-67 however any combination of these sensors 63-67 can be provided while others are not included in the wearable device 6.

Furthermore, the controller 62 may be connected with a memory 68 to store program code to be executed by the controller and to store information about body characteristics and with a power supply 69 to supply power to operate the controller 62 and the sensors 63-67 for acquiring body characteristics. The power supply 69 can be charged by means of a power plug 70 and/or by means of an antenna for wireless charging.

The sensors 63-67 may include
- a microphone 63 for detecting a snoring noise made by the sleeping individual 2,
- an inertia sensor 64 to detect vibrations and/or an orientation of the wearable device and so of the body, particularly the head, neck or chest of the sleeping individual 2,
- a temperature sensor 65 to detect the body temperature of the sleeping individual,
- a blood sensor 66 to detect the blood oxygen level and/or a heart rate;
- a pressure sensor 67, which may be arranged directly or via the air pipe 8 with an airflow through the nose 22 of the sleeping individual 2, so that the respiratory activity of the sleeping individual 2 can be detected.

In one embodiment, the wearable device 6 may be connected to the head or neck of the sleeping individual 2 and may include as a combination of sensors the microphone 63, the inertia sensor 64 and the pressure sensor 67. Such an arrangement has an advantage that a snoring noise can be detected and by means of the pressure sensor 67 the breathing activity can be monitored so that an airways disorder event can be determined by the controller 62 or by the control unit 4. Furthermore, by means of the inertia sensor 64, an orientation of the head 21 can be determined so that the actuating of the active layer 31 in the head support 3 can be made depending on the actual orientation of the head 2. If the inertia sensor 64 has a sufficient sensitivity vibrations caused by snoring could be detected and so the microphone can be omitted.

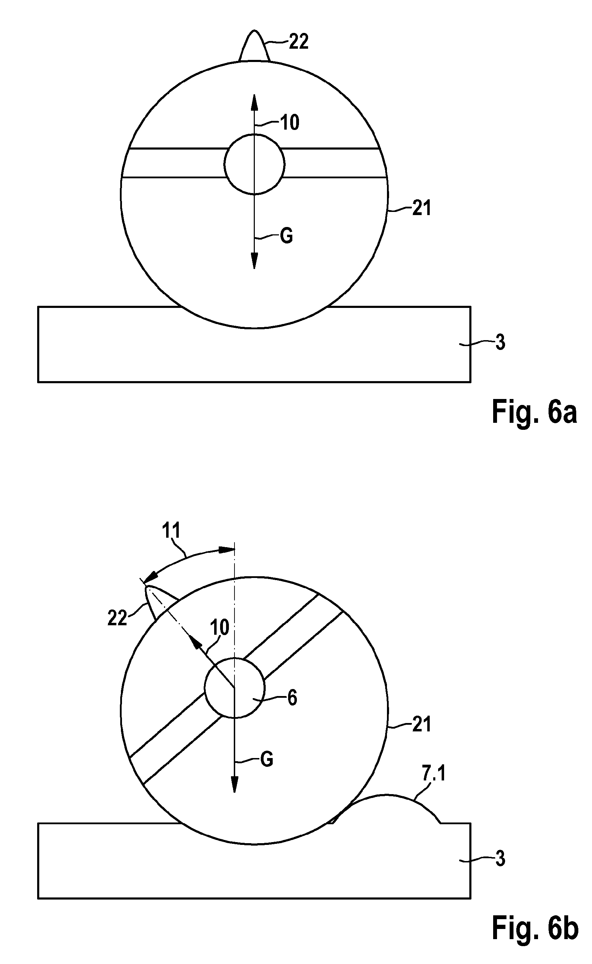

Furthermore, by means of the inertia sensor 64, a turning of the head 21 due to an actuation of the head support 3 can be monitored so that a feedback can be obtained by which angle the head 21 of the sleeping individual 2 resting on the head support 3 is/has been tilted. This is illustrated in Figures 6a and 6b for two different orientations of the head of the sleeping individual 2. The inertia sensor 64 measures its own spatial orientation due to the direction it is exposed to gravity G. The detection of the orientation of the head 21 of the sleeping individual 2 allows to determine the direction into which the head 21 of the sleeping individual 2 has to be turned and the angle by which the head 21 is turned so that the movement of the head 21 can be fully controlled achieved after a respiratory disorder event has been detected.

Furthermore, the sensor data obtained by the inertia sensor 64 can be used to verify that a snoring detected by the sensor data of the microphone 63 origins from the same individual which wears the wearable device 6.

Furthermore, it is also possible that the controller 62 performs an analysis of the data provided by one or more of the sensors 63-67 and uses the communication unit 61 to really transmit corresponding respiratory disorder event information about a respiratory disorder event which is detected as upcoming or ongoing to the control unit 4. So the control unit 4 merely needs to activate the active layer 31 in the head support 3 depending on received information.

Furthermore, the communication unit 61 may provide the information about the body characteristics as detected by one or more of the at least one sensor 63-67 via a suitable transmission protocol, such as WiFi, Bluetooth or the like to the control unit 4 of the head support 3. Hence, it may be provided that the communication unit 61 connects to a portable device, such as a mobile device, via a standard transmission protocol, such as WiFi, Bluetooth or the like, which monitors the information of body characteristics so that the sleeping individual 2 can obtain a timely characteristics about information of the body characteristics during the sleeping time via an app carried out on the mobile device.

## Claims

1. Wearable device (6, 6a, 6b, 6c) for detecting body characteristics and for use in an airways disorder alleviating system, comprising:
- a device housing ;
- a means (7) for attaching the device housing to a body part of a sleeping individual (2);
- one or more sensors (63-67) configured to detect body characteristics and
- a communication unit (61) configured to communicate at least one of information of body characteristics and a respiratory disorder event information indicating that a respiratory disorder event is ongoing or upcoming to an external device.

2. Wearable device (6, 6a, 6b, 6c) according to claim 1, wherein the means for attaching (7) are configured to allow the device housing to be attached to at least one of an upper side (P1) of the head of the sleeping individual, a position (P2) at a forehead of the sleeping individual, a position (P3) at a neck of the sleeping individual, a location (P5) between the nose and the mouth, a position (P4) at a chest of the sleeping individual (2), and a position (P5) aside the nose, wherein the means (7) for attaching particularly comprise at least one of a belt, a strap and a stripe.

3. Wearable device (6, 6a, 6b, 6c) according to claim 1 or 2, wherein the information of body characteristics comprises at least one of:
- a snoring noise,
- vibrations and/or an orientation of the device housing,
- a body temperature of the sleeping individual,
- a blood oxygen level and/or a heart rate,
- a pressure sensor related to a respiratory activity of the sleeping individual.

4. Wearable device (6, 6a, 6b, 6c) according to claim 1 or 2, wherein the one or more sensors comprise one or more of:
- a microphone (63) configured to detect a snoring noise,
- an inertia sensor (64) configured to detect vibrations and/or an orientation of the device housing,
- a temperature sensor (65) configured to detect the body temperature of the sleeping individual (2),
- a blood sensor (66) configured to detect a blood oxygen level and/or a heart rate,
- a pressure sensor (67) configured to detect a respiratory activity of the sleeping individual.

5. Wearable device (6, 6a, 6b, 6c) according to any of the claims 1 to 4, wherein a pressure sensor (40, 67) is connected via a nasal air pipe (8) to the nose (22) of the sleeping individual.

6. Wearable device (6, 6a, 6b, 6c) according to any of the claims 1 to 4, wherein a longitudinal tube (41) is arranged in parallel to a sensing surface of a pressure sensor unit (43) of the pressure sensor (40) so that an opening (42) at a first end of the tube (41) is disposed at the sensing surface, wherein a second end of the tube (41) is disposably attachable to the nasal air pipe (8).

7. Wearable device (6, 6a, 6b, 6c) according to any of the claims 1 to 6, wherein an inertia sensor (64) is provided to detect an orientation of the head or body of the sleeping individual (2) wherein the communication unit (61) is configured to communicate an information about the orientation to an external device.

8. Wearable device (6, 6a, 6b, 6c) according to claim 7, wherein an airflow sensor is provided to detect a respiratory activity of the sleeping individual.

9. Head support (3) comprising:
- a resting surface (32) for a head of a sleeping individual (2) to rest on;
- an active layer (31) configured to allow a separate deformation of the resting surface (32) in sections;
- a control unit (4) configured to receive at least one of information of body characteristics and to determine a respiratory disorder event information indicating that a respiratory disorder event is ongoing or upcoming based on the at least one of information of body characteristics and to actuate the active layer (31) depending on the at least one of information of body characteristics.

10. Head support (3) according to claim 9, wherein the control unit configured to receive an information about the orientation of the head or the body of the sleeping individual as the at least one of information of body characteristics and to actuate the active layer (31) depending on the information about the orientation of the head or the body.

11. Head support (3) according to claim 9 or 10, wherein the control unit (4) configured to receive an information about a breathing activity of the sleeping individual as the at least one of information of body characteristics and to actuate the active layer (31) depending on the information about the breathing activity.

12. An airways disorder alleviating system (1) comprising a wearable device (6, 6a, 6b, 6c) according to any of the claims 1 to 8 and a head support (3) according to any of the claims 9 to 11.
